# EUROPEAN PATENT APPLICATION

(11) **EP 1 712 541 A1**
(43) Date of publication of application: **18.10.2006**
(21) Application number: 05708101.0
(22) Date of filing: 13.01.2005
(51) Int. Cl.: C07C 69/003, C07H 13/06, C07B 41/12

(54) **METHOD OF PREPARING MONOESTERS OF POLYHYDROXYL ALCOHOLS**

(30) Priority: 21.01.2004 ES 200400231
(71) Applicant: CONSEJO SUPERIOR DE INVESTIGACIONES CIENTIFICAS (CSIC), 28006 Madrid (ES); UNIVERSIDAD POLITECNICA DE VALENCIA, 46022 Valencia (ES); Universiti Malaya, Kuala Lumpur 50603 (MY)
(72) Inventor: CORMA CANOS, Avelino, Inst. Tecn. Quimica, E-46022 Valencia (ES); IBORRA CHORNET, Sara, Inst. Tecn. Quimica, E-46022 Valencia (ES); VELTY, Alexandra Isabelle, Inst. Tecn. Quimica, E-46022 Valencia (ES); BEE ABD HAMID, Sharifa, KUALA LAMPUR, 50603 (MY)
(74) Representative: Ungria Lopez, Javier
(86) International application number: PCT/ES2005/070003
(87) International publication number: WO 2005/070865

(57) **Abstract**

The invention relates to a method of preparing monoesters of polyhydroxyl alcohols. The inventive method comprises a first step consisting in protecting the hydroxyl groups of a polyhydroxyl alcohol by means of acetalisation and a second step comprising the sterification of said acetalised polyhydroxyl alcohol with a fatty acid in the presence of one or more solid acid catalysts.

## Description

### Field of the Art

This invention relates to the preparation of polyhydroxyl alcohol monoesters by means of a method comprising a first step of acetalisation of the alcohol and a second step of esterification of the polyhydroxyl alcohol with a fatty acid.

### State of the art

Esters of fatty acids and polyhydroxyl alcohols such as esters of sorbitol (SFAE) are non-ionic surfactants widely used as emulsifers (Span, Tween) and stabilisers in the food and cosmetics industry. Chemically, esters of sorbitol are complex mixtures of esters of various polyols derived from sorbitol. As well as sorbitol, two known polyols are its anhydride (1,4-sorbitan) and its dianhydride (1,4-3,6-isosorbide). Fatty acids of sorbitol can be prepared by a large variety of methods. These methods include:
a) direct esterification of the sorbitol with the fatty acid. This reaction can be conducted in the presence of homogenous acid catalysts, such as p-toluenesulphonic acid, at temperatures between 140-180 °C (Giacometti J. et al., React. Kinet. Catal. Lett. 59 (1996), 235), basic catalysts such as KOH or NaOH at high temperatures (200-240 °C) or mixtures of an acid and a base, particularly NaOH and phosphoric acid (WO-9804540); esterification catalysed by enzymes has also been described b) Acylation of the sorbitol with chloride or anhydride of a fatty acid c) Transesterification of the sorbitol with methyl esters, ethyl esters of a fatty acid or glyceric esters.

In general, the methods described above for the preparation of esters of fatty acids with sorbitol promote the dehydration of the polyalcohol in order to give rise to the ether form, and the products deriving from this process are not really esters of sorbitol and are therefore more correctly identified as esters of sorbitan and/or isosorbide. Depending on the synthesis method, the final mixture contains different degrees of substitution of the hydroxyl groups and different proportions of esters of sorbitol anhydrides. For example, when the esterification is carried out by acylation of sorbitol using the chloride of the fatty acid the product contains small amounts of the esters of sorbitol anhydrides. Nevertheless, direct esterification and the transesterification process lead to a high proportion of esters of sorbitol in the form of anyhdrides. In the literature, methods have been described for the production of esters of sorbitol which provide for the formation of sorbitol anhydrides, nevertheless these methods imply the use of highly toxic solvents such as dimethylformamide, dimethylsulphoxide or pyridine (US-2997492). The residual quantities of these solvents and decomposition products remain in the esters of the sorbitol rendering these products unfit for food applications.

Other disadvantages entailed by the foregoing processes include the use of high temperatures which can have adverse effects on the organic reagents, the use of solvents and the use of homogenous catalysts, which afterwards have to be neutralised. In this regard, the replacement of homogenous catalysts with heterogenous ones offers known advantages, not just in the design of the process (easy separation of the reaction products from the catalyst thereby avoiding neutralisation and extraction processes, and thus reducing the formation of residues and permitting the catalyst to be recycled), but also improving the yield and the selectivity of the desired product by means of the design of a suitable catalyst for a particular process. Nevertheless, few examples have been described in the literature in which heterogenous catalysts are used for this type of process, for example an exchanged ionic resin (Ambelyst 15) has been used as an acid catalyst in the esterification of isosorbide with n-octanoic acid giving rise to 2,5-di-n-octanoic acid isosorbide with a yield of 98% (WO-0183488). W.M. Rhijn et al. (Chem. Commun (1998) 317) describe the use of sulphonic acid functionalised in mesoporous materials for the esterification of sorbitol with lauric acid at 112 °C. Under these conditions, the monoester (isosorbide monolaurate) is obtained as the main product, or at longer reaction times the diester of the isosorbide is obtained. The same work describes the use of a Beta zeolite as catalyst, nevertheless, owing to its highly hydrophilic nature, the conversion of the fatty acid is null and the only reaction observed is the degradation of the sorbitol.

The degree of substitution of the hydroxyl groups will determine the final use of the surfactant and can, to a greater or lesser degree, be controlled by the sorbitol / fatty acid molar ratio used in the process. So, in order to obtain a higher proportion of monoesters of sorbitol, an equimolar ratio is used of sorbitol and the fatty acid, nevertheless it is interesting to highlight that the monoesters of sorbitol marketed as monoesters of sorbitan are in fact mixtures of mono-, di- and triesters of sorbitan, which contain a greater concentration of monoesters and hydroxyl values oscillating between 180-200. The hydroxyl value is related to the degree of esterification and etherification of the sorbitol.

In Patent US-3579547 Traxler et al. describe the preparation of mono- and diesters of carboxylic acids of polyhydroxylic and linear aliphatic alcohols (sorbitol and mannitol) by reaction of the carboxylic acid or a short-chain alkyl ester of said carboxyl acid with the acetalised mannitol or sorbitol, in the presence of an alkaline catalyst. The ester of the carboxylic acid of the acetalised alcohol is thereby obtained, which is then hydrolysed by dissolving that ester in a solvent immiscible with water and dispersing it by means of stirring in a aqueous solution of a mineral acid.

The purpose of the present invention is to overcome the drawbacks stated above, by means of a selective process for the preparation of monoesters of polyhydroxyl alcohols by reaction of the acetal of said polyhydroxylic alcohol with a fatty acid catalysed by solid acids.

### DESCRIPTION OF THE INVENTION

Method for the selective preparation of monoesters of fatty acids and polyhydroxyl alcohols, characterised in that it comprises:
- a first step consisting of protecting the hydroxyl groups of a polyhydroxyl alcohol by means of acetalisation and
- a second step of esterification of said acetalised polyhydroxyl alcohol with a fatty acid in the presence of one or more solid acid catalysts.

The first step of acetalisation of the polyhydroxyl alcohol has the aim of protecting the hydroxyl groups of the alcohol.

The heterogenous acid catalysts used are selected from among microporous molecular sieves and salts of heteropolyacids.

Said salts of heteropolyacids are preferably salts of valence +1 metals, preferably alkaline metals.

The heterogenous catalysts that are salts of heteropolyacids preferably have the formula H₃₋ₓMₓPW, in which M is a valence +1 metal, P is phosphorus, W is tungsten and x has a value between 0.1 and 2.9.

In a more preferable manner still, the heterogenous catalysts based on heteropolyacids are salts of phosphotungstic acid of H₃₋ₓMₓO₄₀PW₁₂, in which M is a valence +1 metal and x has a value between 0.1 and 2.9. Said valence +1 metal is preferably an alkaline metal selected from among Li⁺, Na⁺, K⁺, Rb⁺ and Cs⁺.

Among microporous molecular sieves are to be found those with a regular pore size of between 6-14 Å. These molecular sieves are preferably acid zeolites. Among acid zeolites can be mentioned as examples Faujasite (FAU), Mordenite (MOR) , Omega (MAZ) , Ofretite (OFF), ZSM-4 (MFI), Beta (BEA), SSZ-24 (AFI), MCM-22, SSZ-26 and delaminated zeolites, or mixtures thereof. Among delaminated zeolites can be mentioned zeolite ITQ-2, ITQ-6 and mixtures of the two.

The zeolites used in their acid form possess Si/T^{III} ratios in the range 6 to 400, preferably between 10 and 200, where T^{III} is a trivalent metal, such as Al, B, Ga, Fe.

According to the present inventive method, said first step of acetalisation of the polyhydroxyl alcohol can be carried out with a carbonylic compound, and the acetalisation can be conducted by means of homogenous, or preferably heterogenous, catalysis using a solid acid as catalyst. In the event that this step of acetalisation is carried out by means of heterogenous catalysis, the catalyst can be the same for the two steps of the method and said method can then be carried out according to the "one-pot" mode, as illustrated in example 9.

According to the present inventive method, said first step of acetalisation of the polyhydroxyl alcohol can be carried out in accordance with a conventional process in a reactor selected from among a continuous stirred tank type reactor, a discontinuous stirred tank type reactor, a continuous fixed reactor and a fluidised bed reactor where the catalyst is found.

Said first step of acetalisation can be conducted in an inert atmosphere, at pressure selected from between atmospheric pressure and a pressure between 2 and 10 atmospheres. The acetalisation is preferably carried out at a temperature of between 25 and 60 °C, preferably between 25 and 40 °C.

Said first step of acetalisation is preferably carried out with a quantity of catalyst lying between 1 and 20 %, with respect to the mass of polyhydroxyl alcohol.

In an even more preferred manner, said protection by acetalisation is carried out at atmospheric pressure, under an inert atmosphere, for example nitrogen, at a temperature between 25 and 60 °C, preferably between 25 and 40 °C, and in the presence of a heterogenous catalyst, with a quantity of catalyst lying between 1 and 20 % with respect to the mass of polyhydroxyl alcohol.

Said acetalisation step is carried out using carbonylic compounds selected from among non-substituted aldehydes, substituted aldehydes, non-substituted ketones and substituted ketones.

A low molecular weight aliphatic ketone is preferably used. Examples of these compounds are acetone, butanone, 2-pentanone, 3-pentanone, 3-hexanone, formaldehyde, acetaldehyde, propanal, benzaldehyde, etc. The carbonylic compounds preferably used are acetone and butanone.

The molar ratio carbonylic compound : polyhydroxyl alcohol is preferably between 1:1 and 30:1.

Once the acetalisation is complete, the resulting viscous colourless liquid is used as the starting material for carrying out the second step of esterification with the fatty acid.

The second step of the method includes esterification between the acetal of the polyhydroxyl alcohol and a fatty acid. The esterification is preferably done in the absence of solvent. The acid catalyst in the heterogenous phase used in this process is capable of slowly hydrolysing some acetals of the polyalcohol, giving rise to unprotected hydroxyl groups which can react with the fatty acid. During the esterification, water molecules are released so that there exist two coupled reactions: that of the hydrolysis of the acetal and the esterification of the fatty acid with the alcohol generated by means of hydrolysis of the acetal. The purpose of this method is to control the concentration of free hydroxyl groups with the aim of decreasing the rate of formation of diesters, triesters, tetraesters, etc., as well as preventing the internal cyclation of the polyalcohol chain, especially towards the formation of bicyclic ethers of the dianhydride type (diagram).

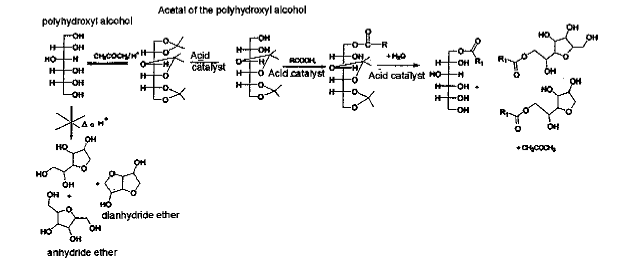

Said second step of esterification can be carried out in accordance with a conventional process in a reactor selected from among a continuous stirred tank type reactor, a discontinuous stirred tank type reactor, or in a continuous fixed reactor or in a fluidised bed reactor where the catalyst is found.

The esterification reaction can be conducted in an inert atmosphere, and the pressure can be atmospheric pressure or it can lie between 2 and 10 atmospheres. The esterification is preferably carried out at a temperature of between 100 and 200 °C, preferably between 100 and 140 °C.

In an especially preferred manner, the esterification reaction is conducted at atmospheric pressure, in an inert atmosphere, for example nitrogen, at a temperature of between 100 and 200 °C, preferably between 100 and 140 °C.

In said esterification step, the molar ratio between the acetalised polyhydroxyl alcohol and the fatty acid preferably lies between 1:1 and 4:1, more preferably it is 1:1.

In the esterification reaction, the quantity of catalyst preferably lies between 1 and 30 % with respect to the total mass of the acetalised polyhydroxyl alcohol.

The catalyst used in this invention is capable of slowly hydrolysing the acetal groups of the acetalised polyalcohol giving rise to unprotected hydroxyl groups which can react with the fatty acid and prevent the formation of high molecular weight esters. The water released in the esterification causes complete hydrolysis of the acetal giving a reaction mixture containing mainly monoesters of the polyhydroxyl alcohol. In the event that the said alcohol is sorbitol, this mixture mainly contains monoesters of the sorbitol and of sorbitan.

According to the present inventive method, the polyhydroxyl alcohols are preferably linear chain aliphatic saturated alcohols with six carbons atoms. Examples of such alcohols are sorbitol, mannitol, iditiol, dulcitol, xylitol and talitol. In an especially preferred manner, the polyhydroxyl alcohol is sorbitol.

The fatty acids used in this invention preferably contain between 6 and 30 carbon atoms, primarily between 8 and 22, and it is also possible to use a mixture of fatty acids. Examples of fatty acids are hexanoic acid (caproic acid), octanoic acid (caprylic acid), decanoic acid (capric acid), dodecanoic acid (lauric acid), tetradecanoic acid (myristic acid), hexadecanoic acid (palmitic acid), octadecanoic acid (stearic acid), eicosanoic acid (arachidic acid), hexadecenoic acid (palmitoleic acid), octadecenoic acid (oleic acid).

The molar ratio between the acetalised polyalcohol and the fatty acid preferably lies between 1:1 and 4:1, preferably 1:1.

The method of selective preparation of the monoesters of polyhydroxyl alcohols, according to the present invention, can also be carried out in "one-pot". This method comprises an acetalisation step, following which the carbonylic compound is eliminated by distillation and then the fatty acid is added to that mixture. According to this embodiment, the same catalyst is capable of carrying out the acetalisation step and the esterification step with the fatty acid.

### EXAMPLES

### Example 1. Preparation of zeolite ITQ-2

Zeolite ITQ-2 was synthesised starting from its laminar precursor MCM-22 (with Si/Al = 15 and 50) following the method described in Corma A., Corell, C., Llopis, F., Martinez, A., and Perez-Pariente, J., Appl. Catal. A, General 115, 121 (1994), and delaminating of the precursor as indicated in Corma, A., Fornes, V., Pergher, S. B., Maesen, Th. L. M., and Buglass, J. G., Nature 376, 353 (1998).

A laminar precursor is prepared starting from a mixture of 0.23 g of sodium aluminate (56% Al₂O₃, 37% Na₂O, Carlo Erba) and 0.8 g of sodium hydroxide (98%, Prolabo) dissolved in 103.45 g of distilled water. Added to this mixture later on are 6.35 g of hexamethyleneimine (HMI) and 7.86 g of silica (Aerosil 200, Degussa), in a consecutive manner. The mixture is stirred vigorously for 30 minutes at room temperature for 11 days in a steel autoclave (PTFE-lined stainless-steel autoclave) at 408 °K under autogenous pressure. The crystalline product obtained was filtered, washed with distilled water to pH < 9, and the filtered solid mass thus obtained was mixed with water as far as obtaining a suspension (slurry) with 20% by weight of the solid.

The delaminating of the solids started from 27 g of the previous suspension (slurry) with 105 g of an aqueous solution of 29 % by weight of hexadecyltrimethylammonium bromide and 33 g of a 40 % aqueous solution of tetrapropylammonium hydroxide for 16 h at 353 °K. The completion of the delamination can be monitored by X-Ray Diffraction, which shows an increase in the distance between laminas from 2.7 nm to 4.5 nm. The laminas are then forced to become displaced by placing the suspension (slurry) in an ultrasound bath (50 w, 40 kHz) for 25 min and 1 h, giving rise to the samples ITQ-2-A and ITQ-2-B, respectively. After adding some drops of an aqueous solution of hydrochloric acid to the solids until a pH of below 2 is reached, the solids are then gathered by centrifugation. The organic matter is then eliminated by roasting the solids at 813 °K to give the zeolites ITQ-2.

### Example 2. Preparation of the catalyst H_{0.5}Cs_{2.5}PW₁₂O₄₀

A solution of Cs₂CO₃ (0.630 g in 15.6 ml of H₂O) was slowly added at a rate of 1 ml/min with magnetic stirring to an aqueous solution formed by mixing 5.15 g of H₃O₄₀PW₁₂ (provided by FLUKA) dissolved in 19.5 ml of water. The addition being completed, the water was evaporated at 40 °C until the dry solid was obtained.

### Example 3. Acetalisation of Sorbitol with acetone

Before commencing the reaction, the catalyst (630 mg, 7 % by weight with respect to sorbitol) was activated by being heated to 200 °C at an approximate pressure of 1 mmHg for 2 h. After that time, the system was allowed to cooling to room temperature and 50 ml of acetone and 9 g (0.05 mols) of sorbitol were added. The resulting suspension was kept under stirring at a temperature of 50 °C for 24 h. At the end of the reaction, the acetone was eliminated from the suspension by means of distillation under vacuum. The residual colourless material with a viscous appearance was used as the starting material for esterification of oleic acid.

### Example 4. Esterification between oleic acid and acetal of sorbitol

An equimolecular mixture of sorbitol acetalised with acetone and oleic acid (molar ratio acetal : oleic acid = 1) was added to the previously activated catalyst (15 % by weight with respect to the total mass of the reagents) and the resulting suspension was subjected to magnetic stirring at 135 °C. At the end of the reaction the catalyst was filtered and washed with dichloromethane and then with methanol. The organic phases were combined, concentrated under vacuum and the organic residue was weighed. The distribution of products of the organic residue was analysed on the basis of a previously weighed sample dissolved in dimethylformamide to which stearic acid was added as an external standard. Table 1 shows the results obtained with the zeolite catalysts Beta, Mordenite, ITQ-2 and H_{0.5}Cs_{2.5}PW₁₂O₄₀.

**Table 1**

| Catalyst (Si/Al) | OH valu e | Time (h) | OA convers ion (%) | Yield (%) ^{a} | | | Molar distribution of esters in the mixture (%) | | |
|---|---|---|---|---|---|---|---|---|---|
| | | | | mono | di | tri | mon o | di | tri |
| MOR (10) | | 24 | 45 | 30 | 15 | 0 | 80 | 20 | 0 |
| | 360 | 48 | 91 | 74 | 17 | 0 | 89 | 11 | 0 |
| BETA (13) | | 8 | 42 | 41 | 1 | 0 | 99 | 1 | 0 |
| | 205 | 24 | 96 | 95 | 1 | 0 | 99 | 1 | 0 |
| ITQ-2 (15) | 185 | 48 | 87 | 67 | 20 | 0 | 86 | 14 | 0 |
| H_{0.5}Cs_{2.5}P | | 8 | 54 | 43 | 11 | 0 | 88 | 12 | 0 |
| W₁₂O₄₀ | 225 | 24 | 80 | 77 | 3 | 0 | 98 | 2 | 0 |

| | | | | | | | | | |
|---|---|---|---|---|---|---|---|---|---|
| Reaction conditions: molar ratio oleic acid / sorbitol = 1; 15 % by weight of catalyst with respect to the total quantity of reagents; temperature: 135 °C. ^{a} Calculated with respect to oleic acid considering the stoichiometric coefficients. | | | | | | | | | |

### Example 5. Esterification between oleic acid and acetal of xylitol using Mordenite as acid catalyst

A mixture of sorbitol acetalised with acetone and oleic acid (molar ratio acetal : oleic acid = 2) was added to the previously activated catalyst (15 % by weight with respect to the total mass of the reagents) and the resulting suspension was subjected to magnetic stirring at 135 °C for 48 h. At the end of the reaction the procedure of example 4 was followed and the results obtained are summarised in Table 2.

**Table 2**

| Catalyst (Si/Al) | Molar ratio xylitol /OA | OA conversion (%) | Yield (%) ^{a} | | | Molar distribution of esters in the mixture (%) | | |
|---|---|---|---|---|---|---|---|---|
| | | | mono | di | tri | mon o | di | tri |
| MOR (10) | 2 | 93 | 74 | 19 | 1 | 88 | 11 | 1 |

| | | | | | | | | |
|---|---|---|---|---|---|---|---|---|
| ^{a}Calculated with respect to oleic acid considering the stoichiometric coefficients. | | | | | | | | |

### Example 6. Esterification between oleic acid and acetal of mannitol

The esterification between oleic acid and mannitol acetalised with acetone was carried out as in example 4 in the presence of the zeolites Mordenite and Beta as acid catalysts (15 % by weight) at a temperature of 135 °C. The results obtained after 48 h of reaction are summarised in Table 3.

**Table 3**

| Catalys t (Si/Al) | Molar ratio xylito I /OA | OA conver sion (%) | Yield (%) ^{a} | | | | Molar distribution of esters in the mixture (%) | | | |
|---|---|---|---|---|---|---|---|---|---|---|
| | | | mon o | di | tr i | tet ra | mon o | di | tr i | tetr a |
| MOR (10) | 2.2 | 79 | 76 | 1 | 2 | - | 99 | 0. 5 | 0. 5 | |
| Beta (13) | 1.3 | 91 | 70 | 2 | 8 | 4 | 90 | 5 | 3 | 2 |

| | | | | | | | | | | |
|---|---|---|---|---|---|---|---|---|---|---|
| ^{a} Calculated with respect to oleic acid considering the stoichiometric coefficients. | | | | | | | | | | |

### Example 7. Esterification between palmitic acid and acetal of sorbitol using xylitol using H_{0.5}Cs_{2.5}PW₁₂O₄₀

The catalyst H_{0.5}Cs_{2.5}PW₁₂O₄₀ (15 % with respect to the total quantity of reagents), previously activated, was added to a mixture of acetal of sorbitol and palmitic acid in a molar ratio sorbitol / palmitic acid = 1. The suspension was heated at 135 °C with magnetic stirring for 9 h. At the end of the reaction the mixture was treated as in example 4. The yields and the molar distribution of esters in the mixture are given in Table 4

**Table 4**

| Catalyst (Si/Al) | Time (h) | PA conversion (%) | Yield (%) ^{a} | | | Molar distribution of esters in the mixture (%) | | |
|---|---|---|---|---|---|---|---|---|
| | | | mono | di | tri | mon o | di | tri |
| H_{0.5}Cs_{2.5}P W₁₂O₄₀ | 9 | 96 | 91 | 4 | 0 | 98 | 2 | 0 |

| | | | | | | | | |
|---|---|---|---|---|---|---|---|---|
| ^{a}Calculated with respect to palmitic acid considering the stoichiometric coefficients. | | | | | | | | |

### Example 8. Esterification of lauric acid and acetal of sorbitol using Mordenite as acid catalyst

The previously activated catalyst (15 % by weight with respect to the total mass of the reagents) was added to a mixture of sorbitol acetalised with acetone and lauric acid in a molar ratio acetal sorbitol / acid = 1.2. The resulting suspension was heated at 135 °C for 48 h. Afterwards, the reaction mixture was treated as in example 4. The results obtained are summarised in Table 5.

**Table 5**

| Catalyst (Si/Al) | Molar ratio sorbi tol /LA | Time (h) | LA conver sion (%) | Yield (%) ^{a} | | | Molar distribution of esters in the mixture (%) | | |
|---|---|---|---|---|---|---|---|---|---|
| | | | | mono | di | tri | mon o | di | tri |
| Mordenit e (10) | 1.2 | 48 | 81 | 70 | 8 | 3 | 86 | 5 | 1 |

| | | | | | | | | | |
|---|---|---|---|---|---|---|---|---|---|
| ^{a} Calculated with respect to lauric acid considering the stoichiometric coefficients. | | | | | | | | | |

### Example 9. Acetalisation of sorbitol and esterification with oleic acid "in one pot"

The catalyst (15 % with respect to the total quantity of reagents), previously activated, was added to a mixture of sorbitol (0.84 g, 4.6 nmol) and acetone (25 ml). The mixture was heated at 60 °C with magnetic stirring until it became homogenous. The acetone was then distilled under vacuum and the oleic acid (1.3 g, 4.6 nmol) was added to the resulting residue which was heated at 135 °C. The reaction having ended, dichloromethane was added and the catalyst was filtered and washed with dichloromethane and then with methanol. The organic phases were combined, concentrated under vacuum and the organic residue was weighed. The distribution of products of the organic residue was analysed on the basis of a previously weighed sample dissolved in dimethylformamide to which stearic acid was added as an external standard. Table 6 shows the results obtained using Mordenite as catalyst.

**Table 6**

| Catalyst (Si/Al) | Time (h) | OA conversion (%) | Yield (%) ^{a} | | | Molar distribution of esters in the mixture (%) | | |
|---|---|---|---|---|---|---|---|---|
| | | | mono | di | tri | mon o | di | tri |
| MOR (10) | 48 | 92 | 77 | 15 | 0 | 91 | 9 | 0 |

| | | | | | | | | |
|---|---|---|---|---|---|---|---|---|
| ^{a}Calculated with respect to oleic acid considering the stoichiometric coefficients. | | | | | | | | |

## Claims

1. Method for the selective preparation of monoesters of fatty acids and polyhydroxyl alcohols, **characterised in that** it comprises:
- a first step consisting of protecting the hydroxyl groups of a polyhydroxyl alcohol by means of acetalisation and
- a second step of esterification of said acetalised polyhydroxyl alcohol with a fatty acid in the presence of one or more solid acid catalysts.

2. Method according to claim 1, **characterised in that** said solid acid catalyst is selected from among microporous molecular sieves and salts of heteropolyacids.

3. Method according to claim 2, **characterised in that** said salts of heteropolyacids are alkaline metal salts.

4. Method according to claim 2, **characterised in that** said salts of heteropolyacids have the formula H₃₋ₓMₓPW, in which M is a valence +1 metal, P is phosphorus, w is tungsten and x has a value between 0.1 and 2.9.

5. Method according to claim 2, **characterised in that** said solid acid catalyst is a salt of phosphotungstic acid of H₃₋ₓMₓO₄₀PW₁₂, in which M is a valence +1 metal and x has a value between 0.1 and 2.9.

6. Method according to claim 5, **characterised in that** said valence +1 metal is an alkaline metal selected from among Li⁺, Na⁺, K⁺, Rb⁺ and Cs⁺.

7. Method according to claim 2, **characterised in that** said microporous molecular sieves are acid zeolites.

8. Method according to claim 7, **characterised in that** said acid zeolites possess a Si/T^{III} ratio, in which T^{III} represents a trivalent metal, between 6 and 400.

9. Method according to claim 7, **characterised in that** said microporous molecular sieve is selected from among a zeolite of the type Faujasite (FAU), Mordenite (MOR), Omega (MAZ), Ofretite (OFF), ZSM-4 (MFI), Beta (BEA), SSZ-24 (AFI), MCM-22, SSZ-26, a delaminated zeolite, or mixtures thereof.

10. Method according to claim 9, **characterised in that** said delaminated zeolite is selected from between zeolite ITQ-2, ITQ-6 and mixtures of the two.

11. Method according to claim 1, **characterised in that** said first step of protection of the hydroxyl groups of a polyhydroxyl alcohol by acetalisation is carried out by means of catalysis selected from between homogenous catalysis or heterogenous catalysis using a solid acid as catalyst.

12. Method according to claim 1, **characterised in that** said first step of acetalisation is carried out in a reactor selected from among a continuous stirred tank type reactor, a discontinuous stirred tank type reactor, a continuous fixed reactor and a fluidised bed reactor where the catalyst is found.

13. Method according to claim 1, **characterised in that** said first step of acetalisation is carried out in an inert atmosphere, at a pressure selected from between atmospheric pressure and a pressure lying between 2 and 10 atmospheres.

14. Method according to claim 1, **characterised in that** said first step of acetalisation is carried out at a temperature of between 25 and 60 °C.

15. Method according to claim 1, **characterised in that** said first step of acetalisation is carried out with a quantity of catalyst lying between 1 and 20 %, with respect to the mass of polyhydroxyl alcohol.

16. Method according to claim 1, **characterised in that** said first step of acetalisation is carried out at atmospheric pressure, under an inert atmosphere of nitrogen, at a temperature between 25 and 40 °C, and in the presence of a heterogenous catalyst, with a quantity of catalyst lying between 1 and 20 % with respect to the mass of polyhydroxyl alcohol.

17. Method according to claim 1, **characterised in that** said first step of acetalisation is carried out using carbonylic compounds selected from among non-substituted aldehydes, substituted aldehydes, non-substituted ketones and substituted ketones.

18. Method according to claim 17, **characterised in that** said ketones are a low molecular weight aliphatic ketone.

19. Method according to claim 17, **characterised in that** said carbonylic compounds are selected from among acetone, butanone, 2-pentanone, 3-pentanone, 3-hexanone, formaldehyde, acetaldehyde, propanal and benzaldehyde.

20. Method according to claim 1, **characterised in that** said first step of acetalisation is carried out using a molar ratio of carbonylic compound : polyhydroxyl alcohol of between 1:1 and 30:1.

21. Method according to claim 1, **characterised in that** said second step of esterification is done in the absence of solvent.

22. Method according to claim 1, **characterised in that** said second step of esterification is carried out in a reactor selected from among a continuous stirred tank type reactor, a discontinuous stirred tank type reactor, a continuous fixed reactor and a fluidised bed reactor where the catalyst is found.

23. Method according to claim 1, **characterised in that** said second step of esterification is carried out in an inert atmosphere, at a pressure selected between atmospheric and a pressure between 2 and 10 atmospheres.

24. Method according to claim 1, **characterised in that** said second step of esterification is carried out at a temperature of between 100 and 200 °C.

25. Method according to claim 1, **characterised in that** in said second step of esterification, the acetalised polyhydroxyl alcohol and the fatty acid are present in a molar ratio of between 1:1 and 4:1.

26. Method according to claim 1, **characterised in that** in said second step of esterification, the catalyst is present in a quantity between 1 and 30 % with respect to the total mass of the acetalised polyhydroxyl alcohol.

27. Method according to claim 1, **characterised in that** said fatty acid contains between 6 and 30 carbon atoms.

28. Method according to claim 1, **characterised in that** said fatty acid is selected from among hexanoic acid, octanoic acid, decanoic acid, dodecanoic acid, tetradecanoic acid, hexadecanoic acid, octadecanoic acid, eicosanoic acid, hexadecenoic acid, octadecenoic acid and mixtures thereof.

29. Method according to claim 1, **characterised in that** said polyhydroxyl alcohol is a linear chain aliphatic saturated alcohol.

30. Method according to claim 1, **characterised in that** said polyhydroxyl alcohol is selected from among mannitol, iditiol, dulcitol, xylitol and talitol.

31. Method according to claim 1, **characterised in that** said polyhydroxyl alcohol is sorbitol.

32. Method according to claim 1, **characterised in that** it is carried out in a "one-pot" reaction, in which said first step of acetalisation and said second step of esterification are conducted without isolation of intermediate products, the fatty acid being added to a mixture resulting from the acetalisation of the first step and the same catalyst acting in the acetalisation step and in the esterification step with the fatty acid.

33. Method according to claim 31, **characterised in that** prior to the addition of the acid and or the fatty acids to the mixture resulting from the acetalisation, a carbonylic compound used **in that** acetalisation step is separated out by distillation.
